# EUROPEAN PATENT APPLICATION

(11) **EP 4 534 642 A1**
(43) Date of publication of application: **09.04.2025**
(21) Application number: 23814088.3
(22) Date of filing: 25.05.2023
(51) Int. Cl.: C12M 3/00, C12N 5/071

(54) **CULTURE VESSEL, PRODUCTION METHOD THEREFOR, AND CULTURE METHOD**

(30) Priority: 30.05.2022 JP 2022087860
(71) Applicant: Mitsui Chemicals, Inc., Tokyo 104-0028 (JP)
(72) Inventor: OKABE Akihiro, Tokyo 104-0028 (JP); MATAYOSHI Tomoya, Sodegaura-shi, Chiba 299-0265 (JP); ESASHIKA Katsuhiro, Sodegaura-shi, Chiba 299-0265 (JP); MATSUGI Tomoaki, Tokyo 104-0028 (JP); KAWADA Jiro, Kawasaki-shi, Kanagawa 212-0032 (JP); TOITA Sayaka, Kawasaki-shi, Kanagawa 212-0032 (JP)
(74) Representative: J A Kemp LLP
(86) International application number: PCT/JP2023/019508
(87) International publication number: WO 2023/234170

(57) **Abstract**

An aspect of the present invention relates to a culture vessel in which from a bottom portion of the culture vessel to which an object to be cultured, such as a cell, attaches, sufficient oxygen for growth of the cell, for example, is supplied, the culture vessel has a culture space suitable for three-dimensionally controlling the growth of the object to be cultured, and the grown object to be cultured can be taken out without damage.

The culture vessel is a culture vessel having a culture space for culturing an object to be cultured, the culture vessel including: a vessel member (B) having an inverted recess portion on a lower surface of a bottom portion, and a first through-hole at one end of the inverted recess portion; and a film member (A) having oxygen permeability, wherein the film member (A) and the vessel member (B) are peelably bonded, the inverted recess portion and the film member (A) form a space α in a state where the film member (A) and the vessel member (B) are bonded, the first through-hole located at one end of the space α receives the object to be cultured, and the space α is a part of the culture space.

## Description

### Technical Field

An aspect of the present invention relates to a culture vessel, a method for producing the culture vessel, and a culture method.

### Background Art

Cells, tissues or organs (hereinafter, also referred to as cells, for example) can be cultured only under conditions suitable for growth, and therefore are required to be placed in a culture vessel such as a dish, a plate or a flask together with a culture medium containing appropriate nutrients, with the temperature, the humidity and the gas concentration of the culture vessel being maintained at predetermined levels.

Further, for efficiently performing the culture, oxygen must be sufficiently and properly supplied. In a culture vessel made from a material having low gas permeability, such as glass or polystyrene, gas supply to the inside of the culture vessel is required, and therefore gas supply to the inside of the vessel from the inside of an incubator by providing an opening on an upper portion of the culture vessel such as a cap or a lid.

However, normally, cells, for example, are often attached to the upper surface of a bottom portion of a culture vessel, or suspended in the vicinity of the bottom surface. In general, the upper surface of a bottom portion of a culture vessel is covered with a culture medium, and therefore the oxygen diffusion rate in the culture medium is limited. In particular, a problem that oxygen supply to cells, for example, to the bottom portion of a culture vessel is not sufficient and proliferation of the cells, for example, is hindered has been known for a long time (Non Patent Literature 1).

Heretofore, cells, for example, cultured *in vitro* (outside the body) using a culture vessel have been used in evaluation of, for example, drug efficacy and safety for development of a drug to rapidly proceed, for example.

Cells, for example, cultured *in vitro* using a culture vessel are required for iPS drug development in which abnormal cells are reproduced using iPS cells and a therapeutic drug is found, and regenerative medicine in which tissues in a living body which have been affected by, for example, transplantation of cells are regenerated. An advanced culture technique is desired in which growth of cells, for example, in a living body can be reproduced *in vitro* (outside the body).

However, in a common culture vessel, there has been a problem that since cells, for example, are two-dimensionally cultured on a plane, the form of cultured cells, for example, considerably differs from the form in a living body. Specifically, there has been a problem that cells, for example, cultured two-dimensionally on a plane are in a flat and elongated form. To address this, development of a technique for three-dimensional culture which enables reproduction of a form of cells, example, in a living body has proceeded.

For example, a cell culture technique in which a three-dimensional structure as an aggregate called a spheroid is prepared by attaching cells, like a bio 3D printer, is known (Non Patent Literature 2).

Development of a technique related to a culture vessel which is made by fine processing and so called a microchannel also proceeds. For example, a technique for culturing neural cells is known in which using cell culture chips, an axon bundle extending from a neural cell body is rapidly grown *in vitro* (Patent Literature 1).

A cell culture vessel having a shape suitable for cell culture, in which a microchannel portion of the culture vessel is detachably attached, so that the culture vessel can be reused by exchanging the microchannel portion has been developed (Patent Literature 2).

Further, a technique related to a culture vessel in which a plurality members are bonded to three-dimensionally control microchannels. The technique is a technique in which channels are formed on a surface of a plastic substrate, and a plastic substrate that has been separately prepared is bonded with an adhesive to prepare a microchannel device (Patent Literature 3).

Since use of an adhesive for a microchannel device causes inhibition of culture, development of a technique for preparing a microchannel device by bonding plastic substrates by thermal compression bonding without use of an adhesive is being made (Patent Literature 4).

### Citation List

### Patent Literature

[Patent Literature 1] WO2017/187696
[Patent Literature 2] WO2006/123570
[Patent Literature 3] JP 2002-139419
[Patent Literature 4] JP 2016-203291

### Non Patent Literature

[Non Patent Literature 1] Stevens, K. M., Oxygen requirements for liver cells in vitro., Nature, 206, 199 (1965)
[Non Patent Literature 2] Journal of Printing Science and Technology, Vol. 51, No. 1, p. 11-17

### Summary of Invention

### Technical Problem

In the technique of Patent Literature 1, a sophisticated organoid having a function similar to that of tissues in a living body and having a controlled form can be prepared by three-dimensionally controlling a culture space with cell culture chips, but when cultured cells are taken out from a narrow culture space, it is necessary to take out the cells from the exit of a microchannel by suction with a pipette, and the cultured cells may be damaged. In addition, there is a problem that the physical load on the cultured cells is heavy.

The technique of Patent Literature 2 is advantageous in taking out cultured cells, but is not suitable for three-dimensionally controlling the growth of the cells because of the structure in which the channel is opened on the upper side. Further, there is the problem of having a structure in which the cells during culture adhere to the bottom surface of a culture vessel, and oxygen cannot be sufficiently supplied from the bottom surface. If oxygen cannot be sufficiently supplied to cells during culture, the cells may grow in a distorted form, or peel from the culture vessel.

In the techniques of Patent Literature 3 and 4, there is an advantage of being able to three-dimensionally control a culture space, but a plurality of members bonded cannot be peeled, and therefore when cultured cells are taken out, the cultured cells may be damaged.

The inventors of the present invention have thought that for culturing an organoid having a function similar to that of tissues in a living body, it is important to develop a dedicated culture vessel which enables simulation of growth of cells in the living body.

Accordingly, an object of an aspect of the present invention is to provide a culture vessel in which from a bottom portion of the culture vessel to which an object to be cultured, such as a cell, attaches, sufficient oxygen for growth of the cell, for example, is supplied, the culture vessel has a culture space suitable for three-dimensionally controlling the growth of the object to be cultured, and the grown object to be cultured can be taken out without damage.

### Solution to Problem

The present inventors have conducted diligent research in order to solve the problems described above. As a result, the present inventors have found that the problems described above can be solved by the following culture vessel, method for producing the culture vessel, and culture method, leading to completion of the present invention. An aspect of the present invention is, for example, the following [1] to [26].

[1] A culture vessel having a culture space for culturing an object to be cultured, the culture vessel comprising: a vessel member (B) having an inverted recess portion on a lower surface of a bottom portion, and a first through-hole at one end of the inverted recess portion; and a film member (A) having oxygen permeability, wherein the film member (A) and the vessel member (B) are peelably bonded, the inverted recess portion and the film member (A) form a space α in a state where the film member (A) and the vessel member (B) are bonded, the first through-hole located at one end of the space α receives the object to be cultured, and the space α is a part of the culture space.
[2] The culture vessel according to [1], wherein the space α is a channel.
[3] The culture vessel according to [1] or [2], wherein a width of the channel is within a range of 1 µm to 1,000 µm, and a depth of the channel is within a range of 1 µm to 1,000 µm.
[4] The culture vessel according to [2] or [3], wherein a length of the channel is within a range of 1 µm to 100 µm.
[5] The culture vessel according to any one of [1] or [4], wherein a thickness of the film member (A) is within a range of 1 µm to 1,000 µm.
[6] The culture vessel according to any one of [1] to [5], wherein the inverted recess portion is an inverted recess portion for a channel.
[7] The culture vessel according to any one of [1] to [6], wherein a peel strength between the film member (A) and the vessel member (B) in a 90° peel test is within a range of 0.01 to 3.0 N/cm as measured in accordance with-K 6854-1: 1999.
[8] The culture vessel according to any one of [1] to [7], wherein a total light transmittance of the film member (A) is 80% or more as measured in accordance with-K-7361-1.
[9] The culture vessel according to any one of [1] or [8], wherein an oxygen permeability of the film member (A) at a temperature of 23°C and a humidity of 0% is within a range of 4,500 to 90,000 cm³/(m²·24h·atm).
[10] The culture vessel according to any one of [1] to [9], wherein the vessel member (B) contains a resin.
[11] The culture vessel according to [10], wherein the resin is a thermoplastic resin.
[12] The culture vessel according to [11], wherein a glass transition point of the thermoplastic resin is 120°C or lower as measured in accordance with-K 7121: 1987.
[13] The culture vessel according to any one of [1] or [12], wherein the film member (A) contains a 4-methyl-1-pentene polymer (X).
[14] The culture vessel according to any one of [1] or [13], wherein the vessel member (B) contains a 4-methyl-1-pentene polymer (X).
[15] The culture vessel according to [13] or [14], wherein the 4-methyl-1-pentene polymer (X) is at least one polymer selected from a 4-methyl-1-pentene homopolymer (X1) and a copolymer (X2) of 4-methyl-1-pentene and at least one olefin selected from ethylene, propylene, and an α-olefin having 4 to 20 carbon atoms.
[16] The culture vessel according to any one of [1] or [15], wherein a water contact angle of at least a culture surface of any of the film member (A) and the vessel member (B) is 50° to 100°.
[17] The culture vessel according to any one of [1] or [16], wherein at least the culture surface of any of the film member (A) and the vessel member (B) is a culture surface coated with at least one material selected from the group consisting of a natural polymer material, a synthetic polymer material and an inorganic material.
[18] The culture vessel according to any one of [1] or [17], wherein at least the culture surface of any of the film member (A) and the vessel member (B) is a hydrophilization-treated culture surface.
[19] The culture vessel according to any one of [1] to [18], wherein a second through-hole is provided at the other end of the inverted recess portion, and the other end of the space α communicates with the second through-hole.
[20] The culture vessel according to any one of [1] to [19], wherein the vessel member (B) includes a liquid storage portion that stores a culture medium, and the liquid storage portion, the first through-hole, the second through-hole and the space α communicate.
[21] The culture vessel according to any one of [1] to [20], wherein the object to be cultured is a neural cell or a cardiac muscle cell.
[22] The culture vessel according to [21], wherein the first through-hole is a first chamber portion that receives the object to be cultured, the first chamber portion receives a cell body of the neural cell, and the space α receives an axon bundle extending from the cell body.
[23] A method for culturing a cell, tissue or organ, comprising a step (I) of incubating a cell, tissue or organ in the culture vessel according to any one of [1] to [22].
[24] The culture method according to [23], comprising a peeling step (II) of peeling the film member (A) from the vessel member (B) after the step (I), and a taking-out step (III) of taking out the incubated cell, tissue or organ after the peeling step (II).
[25] A method for producing the culture vessel according to any one of [1] to [22], comprising a bonding step of peelably bonding a vessel member (B) having an inverted recess portion on a lower surface of a bottom portion and a first through-hole at one end of the inverted recess portion, and a film member (A) having oxygen permeability.
[26] The method for producing a culture vessel according to [25], wherein the bonding step comprises a step of performing hydrophilization treatment on a part or a whole of a bonded surface of the film member (A) and the vessel member (B), and then hot-pressing the hydrophilization-treated bonded surface, or a laser irradiation step of performing laser irradiation in a state where the bonded surface of the film member (A) and the bonded surface of the vessel member (B) are in contact with each other.

### Advantageous Effects of Invention

According to an aspect of the present invention, it is possible to provide a culture vessel in which from a bottom portion of the culture vessel to which an object to be cultured, such as a cell, attaches, sufficient oxygen for growth is supplied, the culture vessel has a culture space suitable for three-dimensionally controlling the growth of the cell, for example, and the cultured cell, for example, can be taken out without damage by peeling the members after the culture.

According to an aspect of the present invention, it is possible to provide a culture vessel in which a cell, tissue or organ is cultured in an environment representing the inside of a living body, and the cultured cell, tissue or organ can be taken out without damage, a method for producing the culture vessel, and a culture method.

### Brief Description of Drawings

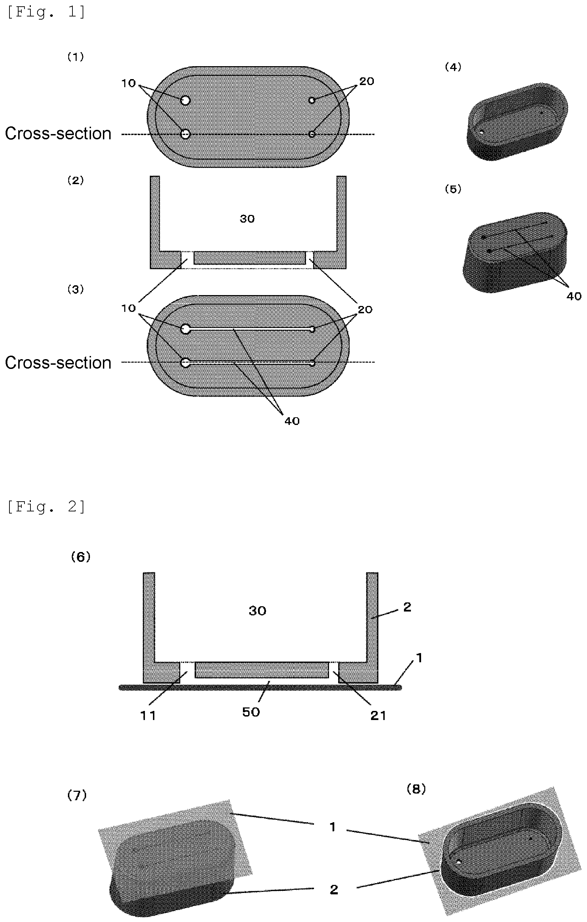

[Figure 1] Figure 1 shows plan views of an upper surface, a cross-section and a lower portion of a bottom surface of a preferred example of a vessel member (B), and three-dimensional views of the vessel member (B) from an upper surface side and a bottom surface side. (1): a plan view of the upper surface, (2): a sectional view of an area shown by a dashed line in (1) and (3), (3): a plan view of the lower portion of the bottom surface, (4): a three-dimensional view from the upper surface side, and (5): a three-dimensional view from the bottom surface side.
[Figure 2] Figure 2 shows an example of a preferred culture vessel in which a film member (A) and a vessel member (B) are peelably bonded. (6): details of a cross-section of the area shown by a dashed line in Figure 1(1) and 1(3), (7): a three-dimensional view from a lower surface side of a bottom portion, and (8): a three-dimensional view from an upper surface side. When an object to be cultured is cultured using a culture vessel according to an aspect of the present invention, use in the state of (8) is preferable.

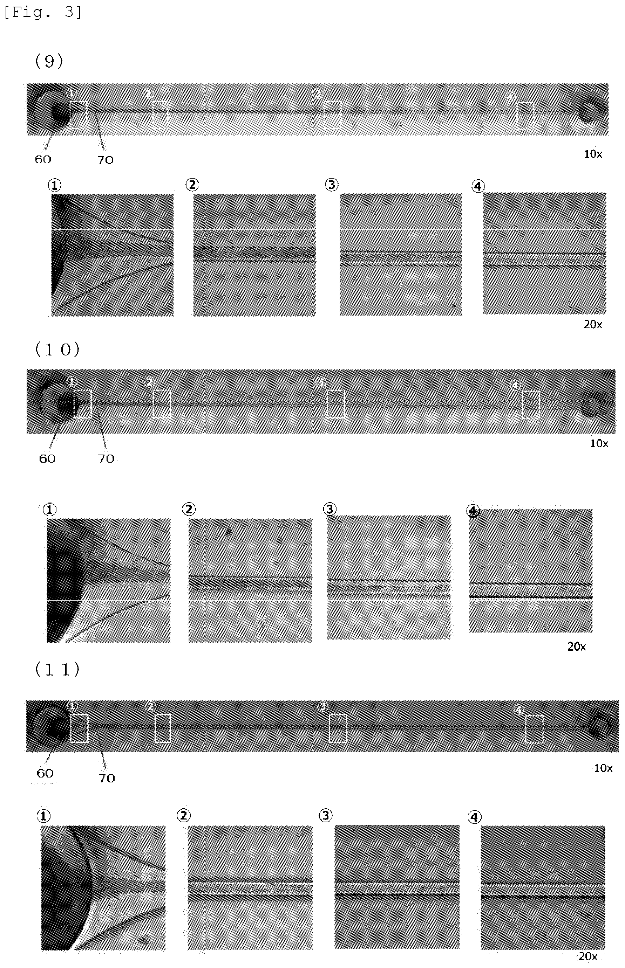

[Figure 3] Figure 3 shows images of an axon bundle of a neural cell (spheroid 1) cultured using culture vessels according to an aspect of the present invention and observed with a light microscope through a film member (A) bonded to a lower surface of a bottom surface of the culture vessel. (9), (10) and (11): images, respectively, of a culture vessel 1, a culture vessel 3 and a culture vessel 4 observed.

### Description of Embodiments

### <Culture vessel>

In an aspect of the present invention, the culture vessel means all vessels that are used for culturing a cell, tissue or organ in a culture medium or a culture solution. Culturing in a culture medium or a culture solution means that culture is performed with at least a part of cells, for example, being in contact with the culture medium or the culture solution, and all cells, for example, to be cultured need not be immersed in the culture medium or the culture solution.

The phrase "an object to be cultured" can be taken out without damage means that there is almost no change in shape of cultured cells, for example, before and after the cultured cells, for example, are taken out from a culture vessel.

A culture vessel according to an aspect of the present invention is a culture vessel having a culture space for culturing an object to be cultured, the culture vessel comprising: a vessel member (B) having an inverted recess portion on a lower surface of a bottom portion, and a first through-hole at one end of the inverted recess portion; and a film member (A) having oxygen permeability, wherein the film member (A) and the vessel member (B) are peelably bonded, the inverted recess portion and the film member (A) form a space α in a state where the film member (A) and the vessel member (B) are bonded, the first through-hole located at one end of the space α receives the object to be cultured, and the space α is a part of the culture space.

A culture vessel according to an aspect of the present invention may also include a member other than the film member (A) and the vessel member (B), for example, a stirring member such as a stirring blade, or a baffle plate for stirring.

### [Film member (A)]

The film member (A) has oxygen permeability. A culture vessel according to an aspect of the present invention is used in a state where the film member (A) and a vessel member (B) described later are bonded.

As a culture vessel according to an aspect of the present invention, a culture vessel including a film member (A) having appropriate oxygen permeability for culturing cells having different oxygen demanding properties can be provided by controlling the oxygen permeability of the film member (A).

In conventional techniques, it is difficult to sufficiently supply oxygen to cells in a culture medium when the cells have a high oxygen demanding property. However, a culture vessel according to an aspect of the present invention, in which a material having high oxygen permeability is selected for the film member (A), thus can be provided as a culture vessel that is suitable even for cells having a high oxygen demanding property.

### (Oxygen permeability)

In an aspect of the present invention, the phrase "having oxygen permeability" means that the oxygen permeability at a temperature of 23°C and a humidity of 0% is a value larger than 0.01 cm³/ (m²·24h·atm).

The oxygen permeability of the film member (A) at a temperature of 23°C and a humidity of 0% is preferably within the range of 0.1 to 1,000,000 cm³/(m²·24h·atm), preferably within the range of 1 to 1,000,000 cm³/ (m²·24h·atm), preferably within the range of 10 to 1,000,000 cm³/(m²·24h·atm), preferably within the range of 100 to 1,000,000 cm³/ (m²·24h·atm), more preferably within the range of 1,000 to 90,000 cm³/ (m²·24h·atm), and still more preferably within the range of 4,500 to 90,000 cm³/ (m²·24h·atm).

When the oxygen permeability is 4,500 to 90,000 cm³/(m²·24h·atm), the oxygen concentration in the culture medium is moderate, so that cells, for example, can sufficiently proliferate, differentiate, and grow. In addition, deterioration of cell functions by oxygen stress can be avoided, which is preferable.

When cells having a high oxygen demanding property, specifically liver cells, kidney cells, cardiac muscle cells and neural cells, are cultured, the oxygen permeability of the film member (A) is preferably within the range of 45,000 to 90,000 cm³/ (m²·24h·atm).

The oxygen permeability can be measured with a differential pressure method gas permeation rate measurement apparatus (Toyo Seiki Seisaku-sho, Ltd.) in accordance with-K-7126-1.

### (Total light transmittance)

In a culture vessel according to an aspect of the present invention, the total light transmittance of the film member (A) is preferably 80% or more, more preferably 85.0% or more, and still more preferably 90.0% or more as measured in accordance with-K-7361-1. The upper limit of the total light transmittance is not defined, but is typically 99.9%.

When the total light transmittance is 80% or more, it can be said that excellent transparency is obtained.

When the total light transmittance is 80% or more, the film member (A) has high transparency, so that an object to be cultured in a culture vessel is easily observed, and cells, for example, are easily observed either with the naked eye or under a microscope, which is preferable.

The thickness of the film member (A) is preferably within the range of 1 µm to 1,000 µm, more preferably within the range of 10 µm to 500 µm, and still more preferably within the range pf 20 µm to 200 µm.

When the thickness of the film member (A) is within the above range, a degree of strength which does not cause damage of the film member (A) can be imparted in peeling, and oxygen suitable for culture can be supplied to an object to be cultured. That is, both the strength and the oxygen permeability of the film member (A) can be secured, which is preferable.

The film member (A) is only required to have a size that allows the film member (A) to be bonded to a vessel member (B) described later, and the size is not particularly limited. From the viewpoint of ease of peeling, the film member (A) is larger in area than the bottom portion of the vessel member (B).

### (Water contact angle)

In the film member (A), the water contact angle of at least the culture surface is preferably 30° or more, more preferably 35° or more, still more preferably 40° or more, and particularly preferably 50° or more, and preferably 120° or less, more preferably 110° or less, and still more preferably 100° or less.

In the film member (A), the water contact angle of at least the culture surface is particularly preferably 50° to 100°.

In an aspect of the present invention, the culture surface means, among portions forming the culture vessel, a portion that is in contact with the culture medium and/or cells, for example, or a portion that is expected to be in contact with the culture medium and/or cells, for example, in culture of cells, for example, as an object to be cultured. That is, the culture surface means a portion that the culture medium inside the culture vessel and/or cells, for example, contact.

When the water contact angle of the film member (A) is adjusted to be in the above range, for example, cells, for example, easily attach to the film member (A), and cells, for example, may easily proliferated uniformly on the film member (A), which is preferable.

In addition, in the case where a natural polymeric material, a synthetic polymeric material or an inorganic material is uniformly applied onto the film member (A), an object to be cultured is easily brought into close contact therewith. After the coating, the natural polymeric material, synthetic polymeric material or inorganic material does not peel during washing with physiological saline and in a cell culture environment, and can be used in cell culture while maintaining a stable initial state, which is preferable.

The method for measuring the water contact angle is not limited, and may be a known method, but is preferably a static drop method. The water contact angle can be measured in accordance with JIS-R3257 (Testing method of wettability of glass substrate).

### (Testing method of wettability of glass substrate)

Measurement can be performed by a method in which in accordance with JIS-R3257 (Testing method wettability of glass substrate), a water droplet with a volume of 4 µL or less which can be considered to have a spherical shape under constant temperature and humidity conditions of 25 ± 5°C and 50 ± 10% is dropped to a surface of a measurement sample, and by a static drop method, the angle of a contact interface between the measurement sample and the water droplet within 1 minute immediately after the water droplet comes into contact with the surface of the measurement sample is measured.

The film member (A) is not particularly limited as long as it has oxygen permeability. The material for the film member (A) is preferably excellent in moldability so that the material can be made into various shapes according to use purposes.

Examples of the material for the film member (A) include resins, carbon materials such as carbon paper, and inorganic materials that can be made porous, such as ceramics. The film member (A) preferably contains a resin among the materials mentioned above. The film member (A) is more preferably a thermoplastic resin.

The thermoplastic resin is not particularly limited, and examples thereof include polyolefin-based resins; polymethacryl-based resins such as polymethyl methacrylate resins; polyacryl-based resins such as polymethyl acrylate resins; polystyrene-based resins; polyvinyl acetal resins; polyvinyl butyral resins; polyvinyl formal resins; polymethylpentene resins; polycarbonate resins; polyether ether ketone resins; polyether-based resins such as polyether ketones; polyester-based resins; polyamide-based resins such as nylon-6, nylon-66 and poly-meta-xylene adipamide; polyamide imide resins; polyimide resins; polyether imide resins; styrene-based elastomers; polyolefin-based elastomers; polyurethane-based elastomers; polyester-based elastomers; polyamide-based elastomers; norbornene resins; polytetrafluoroethylene resins; ethylene tetrafluoroethylene copolymers; polyvinylidene fluoride resins; polyvinyl fluoride resins; thermoplastic polyimide resins; polyvinylidene chloride resins; polyvinyl chloride resins; polyvinyl acetate resins; polysulfone resins; polyphenylene-based resins such as polyphenylene oxide resins and polyphenylene sulfide resins; polysulfone resins; polylactic acid resins; polyether sulfone resins; polyacrylonitrile resins, and styrene-acrylonitrile copolymer resins.

The film member (A) is preferably, among the resins mentioned above, at least one selected from a polyolefin-based resin and a fluorine-based resin, and more preferably a polyolefin-based resin.

Examples of the polyolefin-based resin include homopolymers or copolymers of an α-olefin such as ethylene, propylene, 1-butene, 1-hexene, 4-methyl-1-pentene or 1-octene; high-pressure low-density polyethylene; linear low-density polyethylene (LLDPE); high-density polyethylene; polypropylene; copolymers of propylene and an α-olefin having 2 or more and 10 or less carbon atoms; ethylene/vinyl acetate copolymers (EVAs); and ionomer resins; and fluorine-containing cyclic olefin copolymers.

Examples of the fluorine-based resin include polytetrafluoroethylene (PTFE), polytrifluorochloroethylene, polyvinyl fluoride, polyvinylidene fluoride, dichlorodifluoroethylene, polychlorotrifluoroethylene, fluorinated ethylene-propylene copolymers, perfluoroalkyl vinyl ether polymers, perfluoroalkyl vinyl ester polymers, and ethylene-tetrafluoroethylene copolymers.

Among them, at least one selected from a 4-methyl-1-pentene polymer and polystyrene is more preferable because it is excellent in balance of, for example, moldability, transparency, shape stability, lightweight properties and low-drug-sorption properties, and excellent in peelability between the film member (A) and the vessel member (B), and a 4-methyl-1-pentene polymer (X) described later is still more preferable.

That is, in a culture vessel according to an aspect of the present invention, the film member (A) preferably contains a 4-methyl-1-pentene polymer (X).

The materials for the film member (A) may be contained singly, or two or more thereof may be contained.

### [4-Methyl-1-pentene polymer]

In an aspect of the present invention, 4-methyl-1-pentene homopolymers and copolymers of 4-methyl-1-pentene and other monomers are collectively referred to as a "4-methyl-1-pentene polymer (X)".

In a culture vessel according to an aspect of the present invention, the film member (A) and the vessel member (B) preferably contain a 4-methyl-1-pentene polymer, and more preferably contain a 4-methyl-1-pentene polymer (X).

In a culture vessel according to an aspect of the present invention, the film member (A) and the vessel member (B) may be formed only of a 4-methyl-1-pentene polymer (X), or formed of a composition containing a 4-methyl-1-pentene polymer (X).

The copolymer of 4-methyl-1-pentene and other monomers which is an example of the 4-methyl-1-pentene polymer (X) may be any of a random copolymer, an alternate copolymer, a block copolymer and a graft copolymer. As the copolymer of 4-methyl-1-pentene and other monomers, copolymers of 4-methyl-1-pentene and at least one olefin selected from ethylene and an α-olefin having 3 to 20 carbon atoms (except for 4-methyl-1-pentene) are preferable because they have high strength, are unlikely to break, and are unlikely to warp.

The 4-methyl-1-pentene polymer (X) is preferably at least one polymer selected from a 4-methyl-1-pentene homopolymer, and a copolymer of 4-methyl-1-pentene and at least one olefin selected from ethylene and an α-olefin having 3 to 20 carbon atoms (except for 4-methyl-1-pentene), and more preferably a copolymer of 4-methyl-1-pentene and at least one olefin selected from ethylene and an α-olefin having 3 to 20 carbon atoms (except for 4-methyl-1-pentene).

Examples of the olefin include ethylene, propylene, 1-butene, 1-hexene, 1-heptene, 1-octene, 1-decene, 1-tetradecene, 1-hexadecene, 1-heptadecene, 1-octadecene, and 1-eicosene.

The olefin can be appropriately selected according to physical properties necessary for the film member (A) and the vessel member (B). For example, from the viewpoint of moderate oxygen permeability and excellent rigidity, the olefin is preferably an α-olefin having 8 to 18 carbon atoms, and more preferably at least one selected from 1-octene, 1-decene, 1-dodecene, 1-tetradecene, 1-hexadecene, 1-heptadecene and 1-octadecene. When the number of carbons in the olefin is in the above-described range, the molding processability of the polymer is further improved, and deterioration in appearance due to cracking and breakage of end portions is unlikely to occur in releasing from a roll and a mold during molding. For this reason, the occurrence ratio of defective products of the film member (A) and the vessel member (B) decreases.

The olefins may be contained singly, or two or more thereof may be combined.

From the viewpoint of the strength of the material, the number of carbon atoms is preferably 2 or more, and more preferably 10 or more. In the case where two or more different α-olefins are combined, it is particularly preferable to combine at least one selected from 1-tetradecene and 1-hexadecene and at least one selected from 1-heptadecene and 1-octadecene.

The content of constitutional units derived from 4-methyl-1-pentene in the 4-methyl-1-pentene polymer (X) is preferably 60 to 100 mol%, more preferably 80 to 100 mol%, and still more preferably 85 to 99 mol%.

In the case where the 4-methyl-1-pentene polymer (X) is a copolymer of 4-methyl-1-pentene and at least one olefin selected from ethylene and an α-olefin having 3 to 20 carbon atoms (except for 4-methyl-1-pentene), the content of constitutional units derived from at least one olefin selected from ethylene and an α-olefin having 3 to 20 carbon atoms (except for 4-methyl-1-pentene) in the copolymer is preferably 0 to 40 mol%, more preferably 0 to 20 mol%, and still more preferably 1 to 15 mol%. Note that the content of these constitutional units is based on the amount of all repeating constitutional units in the 4-methyl-1-pentene polymer (X), which is defined as 100 mol%. When the content of the constitutional units is within the above-described range, a culture surface that is excellent in processability and homogeneous is obtained, and warpage is unlikely to occur because the film has well-balanced toughness and strength, which is preferable.

The 4-methyl-1-pentene polymer (X) may have constitutional units other than the constitutional units derived from 4-methyl-1-pentene and the constitutional units derived from the ethylene and an α-olefin having 3 to 20 carbon atoms (hereinafter, also referred to as "other constitutional units") as long as the effects of the present invention are not impaired. The content of other constitutional units is, for example, 0 to 10.0 mol%. In the case where the 4-methyl-1-pentene polymer (X) has other constitutional units, there may be one or more types of constitutional units.

Examples of the monomer from which other constitutional units are derived include cyclic olefins, aromatic vinyl compounds, conjugated dienes, nonconjugated polyenes, functional vinyl compounds, hydroxyl group-containing olefins, and halogenated olefins. As the cyclic olefins, aromatic vinyl compounds, conjugated dienes, nonconjugated polyenes, functional vinyl compounds, hydroxyl group-containing olefins, and halogenated olefins, for example, compounds described in JP2013-169685, paragraphs [0035]-[0041] may be used.

One of the 4-methyl-1-pentene polymers (X) may be used singly, or two or more may be used in combination.

As the 4-methyl-1-pentene polymer (X), a commercially available product may be used. Specific examples thereof include TPX MX001, MX002, MX004, MX0020, MX021, MX321, RT18, RT31 or DX845 (all of which are trademarks). Even a product from another manufacturer can be preferably used as long as it is the 4-methyl-1-pentene polymer (X) satisfying the above-described requirements. One of these commercially available products may be used singly, or two or more thereof may be used in combination.

When a film member (A) and a vessel member (B) according to an aspect of the present invention is formed of a composition containing the 4-methyl-1-pentene polymer (X), components other than 4-methyl-1-pentene polymer (X) may be contained. Examples of the components other than the 4-methyl-1-pentene polymer (X) include additives such as heat resistant stabilizers, light resistant stabilizers, processing aids, plasticizers, antioxidants, lubricants, defoaming agents, antiblocking agents, colorants, modifiers, antibacterial agents, antimold agents, and anti-fogging agents.

The 4-methyl-1-pentene polymer (X) typically has a melting point of 200°C to 240°C and high heat resistance. Since the 4-methyl-1-pentene polymer (X) does not undergo hydrolysis and is excellent in water resistance, boiling water resistance and steam resistance, high-pressure steam sterilization treatment is applicable to the film member (A) and the vessel member (B) containing the 4-methyl-1-pentene polymer (X).

Since the 4-methyl-1-pentene polymer (X) is characterized in that the light transmittance is high (typically 90% or more) and autofluorescence is not emitted, the film member (A) containing the 4-methyl-1-pentene polymer (X) ensures easy observation of an object to be cultured.

Further, since the 4-methyl-1-pentene polymer (X) exhibits excellent resistance to almost all chemicals, and is unlikely to absorb a drug, the 4-methyl-1-pentene polymer (X) does not interfere with the effects of the drug for maintaining an object to be cultured, for example, cells, and are suitably used in drug development screening applications and diagnostic application.

Since the 4-methyl-1-pentene polymer (X) has high strength, a culture vessel including the film member (A) and the vessel member (B) containing the 4-methyl-1-pentene polymer (X) is unlikely to break, and is unlikely to warp.

The 4-methyl-1-pentene polymer (X) can be heat-sealed, and is easy not only to heat-seal to itself but also to heat-bond to another material. In addition, the 4-methyl-1-pentene polymer (X) can be thermally molded, and therefore is easy to mold into a culture vessel with an arbitrary shape, and easy to mold using, for example, an imprinting method or an insert method.

The weight-average molecular weight (Mw) of the 4-methyl-1-pentene polymer (X) measured by the following gel permeation chromatography measurement method (GPC measurement method) is preferably 10,000 to 2,000,000, more preferably 20,000 to 1,000,000, and still more preferably 30,000 to 500,000.

The molecular weight distribution (Mw/Mn) of the 4-methyl-1-pentene polymer (X) as a molecular weight distribution (Mw/Mn) determined by the following GPC method is preferably 1.0 to 30, more preferably 1.1 to 25, and still more preferably 1.1 to 20.

### (GPC measurement method)

In GPC measurement, analysis values (weight-average molecular weight (Mw), number-average molecular weight and Mw/Mn) can be obtained in terms of polystyrene by performing measurement at a temperature of 140°C using ortho-dichlorobenzene as a solvent. The sample concentration in GPC measurement may be, for example, 1.0 to 5.0 mg/mL.

The measurements can be performed under the following conditions. The molecular weight can be determined based on the following conversion method by creating a calibration curve using a commercially available monodisperse polystyrene.
Apparatus: a gel permeation chromatograph Alliance GPC-2000 model (manufactured by Waters Corporation)
Solvent: o-dichlorobenzene
Column: TSKgel column (manufactured by Tosoh Corporation) × 4
Flow rate: 1.0 ml/min
Sample: 0.15 mg/mL o-dichlorobenzene solution
Temperature: 140°C
Molecular weight conversion: in terms of PS/common calibrating method.

For calibration in the common calibration method, the coefficients of the Mark-Houwink equation shown below can be used. Coefficient of polystyrene (PS): KPS = 1.38 × 10-4, aPS = 0.70 Coefficient of polyethylene (PE): KPE = 5.06 × 10-4, aPE = 0.70

The solvent for use in the GPC measurement method is not particularly limited as long as it dissolves the 4-methyl-1-pentene polymer (X). The solvent is preferably ortho-dichlorobenzene.

When the weight-average molecular weight (Mw) is equal to or less than the upper limit, occurrence of defects such as gel is easily suppressed and a film with a uniform surface is easily formed in the case where the film member (A) is produced by melt molding in the above-described method for molding the 4-methyl-1-pentene polymer (X), which is preferable. In addition, solubility in the solvent is further improved, occurrence of defects such as gel is easily suppressed and a film with a uniform surface is easily formed in the case where the film member (A) is produced by a solution-casting method, which is preferable.

When the weight-average molecular weight (Mw) is equal to or less than the upper limit, the vessel member (B) tends to have sufficient strength in the case where the vessel member (B) is produced by an injection molding method in the above-described method for producing the 4-methyl-1-pentene polymer (X). Further, when the molecular weight distribution is within the above-described range, stickiness of the surface of the produced vessel member (B) is easily suppressed, the vessel member (B) tends to have sufficient toughness, and for example, occurrence of bending during molding and cracking during cutting is easily suppressed, which is preferable.

The weight-average molecular weight (Mw) and the molecular weight distribution (Mw/Mn) of the 4-methyl-1-pentene polymers (X), in the case where two or more 4-methyl-1-pentene polymers (X) are used, may be such that Mw and Mw/Mn of each of the two or more 4-methyl-1-pentene polymers (X) are in the above-described ranges.

A culture vessel according to an aspect of the present invention, in which the film member (A) and the vessel member (B) contain the 4-methyl-1-pentene polymer (X), thus may have little adverse effect on culture.

Since the 4-methyl-1-pentene polymer (X) has the above-mentioned excellent characteristics, good shape stability, transparency, sheet shape processability and oxygen permeability are obtained in the case where the film member (A) contains the 4-methyl-1-pentene polymer (X). In addition, good shape stability, transparency and sheet shape processability are obtained in the case where the vessel member (B) contains the 4-methyl-1-pentene polymer (X).

In addition, a culture vessel according to an aspect of the present invention, on which sterilization treatment can be performed, thus is significantly excellent as a culture vessel that is used for culturing cells, for example, as an object to be cultured.

In an aspect of the present invention, the term "polymer" is also used in the meaning that encompasses a homopolymer and a copolymer. Similarly, in an aspect of the present invention, the term "polymerization" is also used in the meaning that encompasses homopolymerization and copolymerization.

The glass transition point of the thermoplastic resin as a material for the film member (A) is preferably 200°C or lower, more preferably 150°C or lower, and still more preferably 120°C or lower as measured in accordance with-K 7121: 1987. A glass transition point of 200°C or lower is preferable because the material is easily molded into fine structures. The lower limit of the glass transition point is not particularly limited, and may be, for example, 0°C or higher.

The surface shape of the film member (A) is not particularly limited. When the film member (A) has an irregular pattern on a surface thereof, the irregular portion can form a part of a space α in bonding of the film member (A) to the vessel member (B).

If a surface obtained by bonding the film member (A) and the vessel member (B) has low smoothness, peel strength may decrease. Thus, it is preferable that the film member (A) have no irregular pattern on a surface thereof.

### [Vessel member (B)]

The vessel member (B) has an inverted recess portion on a lower surface of a bottom portion, and a first through-hole at one end of the inverted recess portion.

The vessel member (B) preferably has a second through-hole at the other end of the inverted recess portion.

That is, when the bottom surface of the vessel member (B) has a first through-hole, and a second through-hole, a culture medium required for culture can be circulated, for example, from the first through-hole to the second through-hole to maintain a constant state of the culture composition at all times. In addition, such a vessel member (B) can also be utilized for cell culture in which two different types of objects to be cultured, such as cells, are seeded in the first through-hole and the second through-hole, respectively, and both the objects to be cultures are joined, which is preferable.

The upper surface of the vessel member (B) is preferably open. When the upper surface is open, supply of a culture medium to a liquid storage portion and removal of the culture medium from the liquid storage portion during culture medium exchange can be easily performed without influencing an object to be cultured, such as cells, which has been cultured.

It is preferable to use the upper surface of the bottom portion of the vessel member (B) as a liquid storage portion that stores a culture medium. When the vessel member has such a structure, the vessel member can be made compact without complicating the structure by separately placing the liquid storage portion.

Preferably, the vessel member (B) includes a liquid storage portion that stores a culture medium, and the liquid storage portion, the first through-hole, the second through-hole and the space α communicate. When the vessel member has such a structure, a culture medium can be circulated, for example, from the first through-hole to the second through-hole through the space α, and the culture medium having a constant composition can be supplied to the space α at all times to maintain constant culture conditions.

The size of the vessel member (B) is not particularly limited, and from the viewpoint of improving ease of handling in, for example, taking out an object to be cultured, for example, the length and the width are each preferably in the range of 0.1 to 200 cm, more preferably in the range of 0.3 to 100 cm, and still more preferably 0.5 to 30 cm.

Since supply of oxygen to the space α is performed by controlling the oxygen permeability of the film member (A), it is desirable that the ratio of the volume of the vessel member (B) to the volume of the space α ([volume of vessel member (B)] / [volume of space α]) be equal to or greater than a certain level so that supply of oxygen from the vessel member (B) to the space α does not have a significant influence. On the other hand, from the viewpoint of volume efficiency as a culture vessel, it is desirable that the ratio of the volume of the vessel member (B) to the volume of the space α be prevented from becoming excessively large.

When these are taken together, the volume ratio of the vessel member (B) to the space α ([volume of vessel member (B)] / [volume of space α]) is preferably 1 to 100,000,000, more preferably 1 to 1,000,000, and still more preferably 10 to 10,000.

### (Inverted recess portion)

The shape of the inverted recess portion of the lower surface of the bottom portion of the vessel member (B) is not particularly limited. Examples of the shape of the inverted recess portion include a semicircular shape, a semi-elliptical shape, a rectangular shape, a square shape, a trapezoidal shape, an inverted V shape, and an inverted U shape in cross-sectional view. In the case where the object to be cultures is a neural cell, the shape of the inverted recess portion is preferably, among the shapes mentioned above, a semicircular shape, an inverted U shape, a trapezoidal shape or a square shape in cross-sectional view because the axon easily elongates along the inverted recess portion, and the object to be cultured, which has been cultured, is easily taken out without damage.

The inverted recess portion of the lower surface of the bottom portion of the vessel member (B) is preferably an inverted recess portion for a channel. In other words, it is preferable that a portion composed of the inverted recess portion of the lower surface of the bottom portion of the vessel member (B) and the film member (A) be used as a channel in a state where the film member (A) and the vessel member (B) are bonded.

Further, the shape of the inverted recess portion for a channel is more preferably any of a circular shape, an inverted U shape, a trapezoidal shape and a square shape in cross-sectional view.

The size of the inverted recess portion of the lower surface of the bottom portion of the vessel member (B) is not particularly limited, and the maximum depth of the inverted recess portion in a perpendicular direction is within the range of preferably 1 to 1,000 µm, more preferably 10 to 1,000 µm, more preferably 20 to 200 µm, more preferably 30 to 180 µm, and still more preferably 50 to 100 µm. The maximum width in a horizontal direction is within the range of preferably 10 to 1,000 µm, more preferably 20 to 200 µm, more preferably 30 to 180 µm, and still more preferably 50 to 100 µm.

The length of the inverted recess portion of the lower surface of the bottom portion of the vessel member (B) in an extending direction is not particularly limited, and is preferably sufficient to store an axon bundle having a desired length when the object to be cultured is a neural cell. The length of the inverted recess portion of the lower surface of the bottom portion in the extending direction is preferably 1 to 50 mm, more preferably 5 to 30 mm, more preferably 5 to 20 m, still more preferably 5 to 15 mm.

The inverted recess portion of the lower surface of the bottom portion of the vessel member (B) is a groove having a semicircular shape or a square shape in cross-sectional view. Particularly preferably, the inverted recess portion has a maximum depth of 50 to 100 µm in the perpendicular direction, a maximum width of 50 to 100 µm in the horizontal direction, and a length of 5 to 15 mm in the extending direction. In the case where the object to be cultures is a neural cell, the above-described shape is particularly preferable because the axon easily elongates along the channel portion, and the object to be cultured, which has been cultured, is easily taken out without damage.

### (First through-hole)

The vessel member (B) has an inverted recess portion on a lower surface of a bottom portion, and a first through-hole at one end of the inverted recess portion.

The first through-hole is a first chamber portion in a state where the film member (A) and the vessel member (B) are bonded. The first chamber portion has a role of, for example, receiving an object to be cultured.

The shape of the first through-hole is not particularly limited, and is preferably a cylindrical shape. The size of the first through-hole is not particularly limited as long as it can receive an object to be cultured. The size of the first through-hole is preferably 0.1 to 10 mm in diameter, more preferably 0.5 to 5 mm in diameter, and still more preferably 0.8 to 2 mm in diameter.

### (Second through-hole)

The vessel member (B) preferably has a second through-hole. The second through-hole is a second chamber portion in a state where the film member (A) and the vessel member (B) are bonded. The second chamber portion has a role of, for example, serving as a vent and a discharge port in circulation of a culture medium.

The shape of the second through-hole is not particularly limited, and is preferably a cylindrical shape. The size of the second through-hole is not particularly limited as long as it can receive an object to be cultured. The size of the first through-hole is preferably 0.1 to 10 mm in diameter, more preferably 0.1 to 5 mm in diameter, and still more preferably 0.3 to 2 mm in diameter.

The second through-hole is only required to enable, for example, efficient ventilation and discharge of a culture medium, and need not be identical in size to the first through-hole. In particular, in the case of use for culture in which it is necessary to secure a sufficient space for the object to be cultured, the first through-hole is preferably large in size than the second through-hole.

The inverted recess portion having a first through-hole at one end thereof is referred to as a module. The module preferably has a second through-hole.

The number of modules is not particularly limited. The vessel member (B) includes preferably 1 to 100 modules, more preferably 1 to 50 modules, and still more preferably 1 to 20 modules.

The vessel member (B) is only required to include a module, and the size and the shape of the vessel member (B) are not particularly limited.

More preferably, the bottom surface of the vessel member (B) has a first through-hole having a diameter of 0.8 to 2 mm, and a second through-hole having a diameter of 0.3 to 2 mm, and each of the numbers of the first through-holes and the second through-holes is 1 to 40.

### (Liquid storage portion)

In the liquid storage portion, a culture medium can be stored in a state where the film member (A) and the vessel member (B) are bonded. The volume of the liquid storage portion is preferably 0.1 to 10,000 mL, more preferably 0.5 to 1,000 mL, and still more preferably 1 to 100 mL. The volume of the culture medium stored is not particularly limited, and is preferably 0.1 to 10,000 mL, more preferably 0.5 to 1,000 mL, and still more preferably 1 to 100 mL.

The liquid storage portion is preferably used as a culture medium storing portion for supplying a culture medium to the space α. When a culture medium is stored in the liquid storage portion, the ratio of the volume of the liquid storage portion to the volume of the space α is required to be equal to or greater than a certain level. On the other hand, when an object to be cultured is taken out from the space α, for example, it is necessary to discharge, for example, a culture medium stored in the liquid storage portion. For this reason, from the viewpoint of ease of handling, it is desirable that the ratio of the volume of the liquid storage portion to the volume of the space α be prevented from becoming excessively large.

When these are taken together, the volume ratio of the liquid storage portion to the space α ([volume of liquid storage portion] / [volume of space α]) is preferably 1 to 100,000,000, more preferably 1 to 1,000,000, and still more preferably 10 to 500,000.

In the liquid storage portion, the first through-hole, the second through-hole and the space α preferably communicate in a state where the film member (A) and the vessel member (B) are bonded.

The material for the vessel member (B) preferably has moldability so that the material can be made into various shapes according to use purposes. In particular, good moldability that can meet fine structures is important. Examples of the material for the vessel member (B) include resins, carbon materials such as carbon paper, and inorganic materials that can be made porous, such as ceramics. The vessel member (B) preferably contains a resin among the materials mentioned above.

Further, it is more preferable that the vessel member (B) contain a resin, with the resin being a thermoplastic resin.

The thermoplastic resin is not particularly limited, and examples thereof include polyolefin-based resins; polymethacryl-based resins such as polymethyl methacrylate resins; polyacryl-based resins such as polymethyl acrylate resins; polystyrene-based resins; polyvinyl acetal resins; polyvinyl butyral resins; polyvinyl formal resins; polymethylpentene resins; polycarbonate resins; polyether ether ketone resins; polyether-based resins such as polyether ketones; polyester-based resins; polyamide-based resins such as nylon-6, nylon-66 and poly-meta-xylene adipamide; polyamide imide resins; polyimide resins; polyether imide resins; styrene-based elastomers; polyolefin-based elastomers; polyurethane-based elastomers; polyester-based elastomers; polyamide-based elastomers; norbornene resins; polytetrafluoroethylene resins; ethylene tetrafluoroethylene copolymers; polyvinylidene fluoride resins; polyvinyl fluoride resins; thermoplastic polyimide resins; polyvinylidene chloride resins; polyvinyl chloride resins; polyvinyl acetate resins; polysulfone resins; polyphenylene-based resins such as polyphenylene oxide resins and polyphenylene sulfide resins; polysulfone resins; polylactic acid resins; polyether sulfone resins; polyacrylonitrile resins, and styrene-acrylonitrile copolymer resins.

The vessel member (B) is preferably, among the resins mentioned above, at least one selected from a polyolefin-based resin and a fluorine-based resin, and more preferably a polyolefin-based resin.

In the case where a polyolefin-based resin and a fluorine-based resin are used as the material for the vessel member (B), a material similar to that for the film member (A) can be used.

As the material for the vessel member (B), at least one selected from a 4-methyl-1-pentene polymer and polystyrene is more preferable because it is excellent in balance of, for example, moldability, transparency, shape stability, lightweight properties and low-drug-sorption properties, and excellent in peelability between the film member (A) and the vessel member (B), and the above-described 4-methyl-1-pentene polymer (X) is still more preferable.

That is, in a culture vessel according to an aspect of the present invention, the vessel member (B) preferably contains a 4-methyl-1-pentene polymer (X).

The materials for the vessel member (B) may be contained singly, or two or more thereof may be contained.

The glass transition point of the thermoplastic resin as a material for the vessel member (B) is preferably 200°C or lower, more preferably 150°C or lower, and still more preferably 120°C or lower as measured in accordance with-K 7121: 1987. A glass transition point of 200°C or lower is preferable because the material is easily molded into fine structures. The lower limit of the glass transition point is not particularly limited, and may be, for example, 0°C or higher.

### (Water contact angle)

In the vessel member (B), the water contact angle of at least the culture surface is preferably 1° to 130°, more preferably 1° to 120°, and still more preferably 1 to 110°.

The water contact angle can be measured in accordance with, for example, Japanese Industrial Standard JIS-R3257 (Testing method of wettability of glass substrate).

### [Method for producing film member (A)]

The method for producing the film member (A) is not particularly limited. For example, in the case of the film member (A) contains a resin, it can be produced by the following method.

Examples of the method for forming the film member (A) include molding processing methods such as injection molding, extrusion, press molding and blow molding. The method for forming the film member (A) is preferably an inflation method and a T-die extrusion method among the methods mentioned above. The film member (A) can be produced using a common method for molding a resin.

In the case where the film member (A) contains the 4-methyl-1-pentene polymer (X), the amount of the 4-methyl-1-pentene polymer (X) is preferably 90% by mass or more and less than 100% by mass, more preferably 95% by mass and less than 100% by mass, and still more preferably 99% by mass or more and less than 100% by mass, in 100% by mass of the film member (A). This is because the inclusion of a large amount of components other than the 4-methyl-1-pentene polymer (X) may cause not only a decrease in oxygen permeability, but also a decrease in transparency or a decrease in strength.

Examples of the component other than the 4-methyl-1-pentene polymer (X) include additives such as heat resistant stabilizers, light resistant stabilizers, processing aids, plasticizers, antioxidants, lubricants, defoaming agents, antiblocking agents, colorants, modifiers, antibacterial agents, antimold agents, and anti-fogging agents.

### [Method for producing vessel member (B)]

The method for producing the vessel member (B) is not particularly limited. For example, in the case of the vessel member (B) contains a resin, it can be produced by the following method.

Examples of the method for forming the vessel member (B) include molding processing methods such as injection molding, extrusion, press molding and blow molding. The method for forming the vessel member (B) is preferably injection molding among the methods mentioned above. The vessel member (B) can be produced using a common method for molding a resin.

In the case where the vessel member (B) contains the 4-methyl-1-pentene polymer (X), the amount of the 4-methyl-1-pentene polymer (X) is preferably 90% by mass or more and less than 100% by mass, more preferably 95% by mass and less than 100% by mass, and particularly preferably 99% by mass or more and less than 100% by mass, in 100% by mass of the vessel member (B). This is because the inclusion of a large amount of components other than the 4-methyl-1-pentene polymer (X) may cause not only a decrease in oxygen permeability, but also a decrease in transparency or a decrease in strength.

Examples of the component other than the 4-methyl-1-pentene polymer (X) include additives such as heat resistant stabilizers, light resistant stabilizers, processing aids, plasticizers, antioxidants, lubricants, defoaming agents, antiblocking agents, colorants, modifiers, antibacterial agents, antimold agents, and anti-fogging agents.

### [Peeling]

The film member (A) and the vessel member (B) are peelably bonded.

The term "peelable" means a state in which the bonded film member (A) and vessel member (B) can be peeled normally by the human hand without significantly impairing either of the shapes of the members. It is only required to enable peeling the film member (A) from the vessel member (B) without damaging an object to be cultured.

When the film member (A) is peeled from the vessel member (B), the peeling can be performed, for example, in a manner such that the hand is held against the members placed with the film member (A) on the bottom surface side, and the vessel member (B) is gripped by the hand, and then held up.

### (Peel strength in peel test)

For whether an object to be cultured, which has been cultured, can be taken out without damage after the bonded film member (A) and vessel member (B) are peeled, for example, the peel strength in a peel test, which is measured in accordance with-K 6854-1: 1999, can be used as an index.

In a culture vessel according to an aspect of the present invention, the peel strength between the film member (A) and the vessel member (B) in a 90° peel test is preferably within the range of 0.01 to 3.0 N/cm, more preferably within the range of 0.05 to 1.0 N/cm, more preferably within the range of 0.05 to 0.5 N/cm, and still more preferably within the 0.1 to 0.5 N/cm as measured in accordance with-K 6854-1: 1999.

The peel strength is preferably 0.01 N/cm or more because the film member (A) is unlikely to peel under its own weight from the vessel member (B). On the other hand, the peel strength is preferably 3.0 N/cm or less because an object to be cultured, which has been cultured, is unlikely to be damaged by impact during peeling. The above-described peel strength is preferable because an object to be cultured, such as a neural cell, is unlikely to be damaged by, for example, impact during peeling which is caused by sound-induced vibration or vibration of the hand holding the culture vessel.

### [Bonding]

The film member (A) and the vessel member (B) are bonded.

In a culture vessel according to an aspect of the present invention, the bonding is preferably performed such that liquid leakage from a portion where the film member (A) and the vessel member (B) are bonded does not occur when the liquid storage portion is filled with ethanol and an aqueous solution such as a culture solution.

In an aspect of the present invention, the bonding means a state in which bonding is performed without using an adhesive.

The bonding can be carried out by pressurization or heating treatment, for example, treatment such as compression bonding, laser irradiation or hot-pressing. From the viewpoint of securing both an ability to peel the members without damage and a degree of adhesion which prevents liquid leakage over a long period of time, it is preferable to carry out bonding by hot-pressing among the treatments mentioned above. The bonding can be carried out by performing pressurization or heating treatment on at least one of the film member (A) and the vessel member (B).

The present inventors have found that when a 4-methyl-1-pentene polymer (X) which enables bonding that ensures high adhesion is used as a material for the film member (A) and the vessel member (B), and the peel strength between the film member (A) and the vessel member (B) in a 90° peel test is in the range of 0.01 to 3.0 N/cm as measured in accordance with-K 6854-1: 1999 so that peeling can be performed without applying a force so strong that the film member (A) and the vessel member (B) are damaged, a more preferred culture vessel is obtained.

### (Surface treatment)

In a culture vessel according to an aspect of the present invention, surface treatment is preferably performed on at least the culture surface of any of the film member (A) and the vessel member (B) to obtain a state in which attachment easily occurs. It is preferable to perform the surface treatment before the film member (A) and the vessel member (B) are bonded.

Examples of the surface treatment include hydrophilization treatment. The method for use in the hydrophilization treatment is not particularly limited, and examples thereof include corona treatment, plasma treatment, ozone treatment, ultraviolet treatment, chemical vapor deposition, etching, or addition of a specific functional group such as a hydroxyl group, an amino group, a sulfone group, a thiol group or a carboxyl group, treatment with a specific functional group, such as silane coupling, and surface roughening with, for example, an oxidant. Among them, surface hydrophilization treatment such as ultraviolet treatment, corona treatment, plasma treatment or ozone treatment is preferably performed because the wettability of the surface of the culture material can be enhanced to efficiently culture cells. One of these surface treatments may be performed singly, or two or more thereof may be performed in combination.

In the case where the surface treatment is performed, it is preferably performed on at least the culture surface. In the case where plasma treatment is performed, as a gas to be entrained, nitrogen, hydrogen, helium, oxygen or argon, for example, is used, and at least one gas selected from nitrogen, helium and argon is preferably selected.

In particular, at least the culture surface of any of the film member (A) and the vessel member (B) is preferably a plasma-treated culture surface because the surface can be brought into a state in which attachment easily occurs, and hydrophilization can be performed sufficiently.

Examples of the plasma treatment include vacuum plasma treatment, normal-pressure plasma treatment, and highpressure plasma treatment. In a culture vessel according to an aspect of the present invention, at least the culture surface of any of the film member (A) and the vessel member (B) is preferably treated by at least one selected from vacuum plasma treatment and normal-pressure plasma treatment, among the plasma treatments mentioned above.

The vacuum plasma treatment can be performed using, for example, a commercially available vacuum plasma surface treatment apparatus (manufactured by Diener electronic GmbH & Co. KG).

The normal-pressure plasma treatment can be performed using, for example, a commercially available normal-pressure plasma surface treatment apparatus (manufactured by Sekisui Chemical Company, Limited).

In the case where normal-pressure plasma treatment is performed, as a gas to be entrained, nitrogen, hydrogen, helium, oxygen or argon, for example, is used, and at least one gas selected from nitrogen, helium and argon is preferably selected.

The surface treatment of the film member (A) and the vessel member (B) is preferably vacuum plasma treatment from the viewpoint of particularly efficiently modifying the surface of the member.

In a culture vessel according to an aspect of the present invention, at least the culture surface of any of the film member (A) and the vessel member (B) is preferably a hydrophilization-treated culture surface.

When hydrophilization treatment is performed on at least the culture surface of any of the film member (A) and the vessel member (B), the wettability of the culture surface is enhanced. In addition, the culture surface of the culture vessel is easily coated with a natural polymeric material, a synthetic polymeric material or an inorganic material.

It is preferable to perform hydrophilization treatment on at least the culture surface of the film member (A) because adhesion between the film member (A) and an object to be cultured is improved, so that the object to be cultured can be uniformly proliferated on the surface of the film member (A).

In a culture vessel according to an aspect of the present invention, at least the culture surface of any of the film member (A) and the vessel member (B) is preferably a culture surface coated with at least one material selected from the group consisting of a natural polymer material, a synthetic polymer material and an inorganic material.

In a culture vessel according to an aspect of the present invention, the coating treatment is preferably performed because the culture vessel does not deform, discolor and peel during washing with physiological saline after the coating treatment and in an environment of culture of an object to be cultured, and the culture vessel can be used while a stable initial state is maintained.

A culture vessel according to an aspect of the present invention is preferably coated with an extracellular matrix protein. When coating treatment is performed with an extracellular matrix protein, the extracellular matrix protein functions as an adhesive molecule of cells, for example, to easily form a scaffold for cell proliferation. The coating treatment can be performed using a commercial product. Although there is no particular limitation, for example, a mouse sarcoma-derived protein (manufactured by Thermo Fisher Scientific, Inc., trade name: Geltrex) and human recombinant-derived laminin (manufactured by Oriental Yeast Co., ltd., trade name: rLAMININ-5) can be used.

### [Culture space]

A culture vessel according to an aspect of the present invention has a culture space.

In a culture vessel according to an aspect of the present invention, it is preferable that in a state where a film member (A) and a vessel member (B) having an inverted recess portion on the lower surface of a bottom portion are bonded, the inverted recess portion and the film member (A) form a space α, the first through-hole located at one end of the space α receive the object to be cultured, the space α be a part of the culture space, a second through-hole be provided at the other end of the inverted recess portion, and the other end of the space α communicate with the second through-hole.

In an aspect of the present invention, the culture space means, for example, a first chamber portion, a liquid storage portion and a space α. A culture vessel according to an aspect of the present invention preferably includes a second chamber portion as a part of the culture space. In addition, as a part of the culture space, another space may be provided in which one end communicates with the first chamber portion or the second chamber portion, and the other end communicates with the space α.

The culture space is preferably a space in which an object to be cultured and a culture solution or a culture medium necessary for culture of the object to be cultured can be brought into contact with each other, or included.

### [Space α]

In an aspect of the present invention, the space α is a part of the culture space. The space α is suitable for three-dimensionally controlling the growth of cells, for example.

In a culture vessel according to an aspect of the present invention, the space α is preferably a channel.

### [Channel]

In an aspect of the present invention, the channel means a tubular space in which an object to be cultured can grow with directionality, and none of both ends are closed so that substances necessary for culture, such as a culture solution, can be circulated. The state in which none of both ends are closed refers to a state in which the channel is not blocked by a solid such as a resin, and is connected to some space through which liquid and gas can be supplied to the space α. Both ends of the channel are opened to a space outside the vessel, such as atmospheric air, or connected to some other gaps formed by the first chamber portion, the second chamber portion, the liquid storage portion or constituent members.

One end and the other end of the channel may be connected to different places. For example, one end may be connected to the first chamber portion, with the other end being connected to the second chamber portion.

In a culture vessel according to an aspect or the present invention, the channel is preferably within the range of 1 µm to 1,000 µm in width and within the range of 1 µm to 1,000 µm in depth, the channel is more preferably within the range of 10 µm to 1,000 µm in width and within the range of 10 µm to 1,000 µm in depth, the channel is more preferably within the range of 10 µm to 200 µm in width and within the range of 10 µm to 200 µm in depth, the channel is more preferably within the range of 20 µm to 200 µm in width and within the range of 20 µm to 200 µm in depth, the channel is more preferably within the range of 30 µm to 180 µm in width and within the range of 30 µm to 180 µm in depth, and the channel is still more preferably within the range of 50 µm to 100 µm in width and within the range of 50 µm to 100 µm in depth.

In a culture vessel according to an aspect of the present invention, the length of the channel is preferably within the range of 1 mm to 100 mm, the length of the channel is more preferably within the 1 mm to 50 mm, the length of the channel is more preferably within the range of 5 mm to 30 mm, the length of the channel is within the range of 5 mm to 20 mm, and the length of the channel is still more preferably within the range of 5 mm to 15 mm.

In a culture vessel according to an aspect of the present invention, the space α is preferably used as microchannels. A culture vessel including microchannels is called a microchannel device or a microchannel chip.

The microchannel device is a generic term of devices for applying fine processing to a culture surface of a culture vessel to provide microchannels and reaction vessels, which are to be applied to biological studies and chemical engineering. Examples of the microchannel device include apparatuses called microTAS (Micro Total Analysis Systems) and Lab on a Chip. These apparatuses are being applied as next-generation culture apparatuses.

### [Object to be cultured]

A culture vessel according to an aspect of the present invention has a culture space for culturing an object to be cultured.

The object to be cultured is a cell, tissue or organ.

In an aspect of the present invention, a cell, tissue or organ is also referred to simply as "cells, for example".

The origin of cells, for example, is not limited, and may be any living organism such as an animal, a plant, an insect, a fungus, a protist or a bacterium, but is preferably an animal or a plant, more preferably an animal, and still more preferably a mammal.

In a culture vessel according to an aspect of the present invention, oxygen necessary for growth is sufficiently supplied to the bottom surface of the space α in which cells, for example, are cultured. In addition, the cells, for example, are preferably cells in that the space α is suitable for three-dimensionally controlling the growth of cells, for example, and that the cultured cells, for example, can be taken out without damage by peeling the film member (A) and the vessel member (B) after the culture.

In an aspect of the present invention, the cells, for example, are not particularly limited, and are preferably aerobic cells, for example, and more preferably cells, for example, that do not include anaerobic cells, for example.

In an aspect of the present invention, the cells may be non-adherent cells or adherent cells, but are preferably adherent cells.

Examples of the adherent cells include fibroblast cells, mesenchymal stem cells, hematopoietic stem cells, neural stem cells, neural cells, corneal epithelial cells, oral mucosal epithelial cells, retinal pigment epithelium cells, periodontal ligament stem cells, myofibroblastic cells, cardiac muscle cells, liver cells, splenic endocrine cells, cutaneous keratinized cells, skin fibroblast cells, subcutaneous fat-derived progenitor cells, kidney cells, basal hair follicle root sheath cells, nasal mucosa epithelial cells, vascular endothelial progenitor cells, vascular endothelial cells, vascular smooth muscle cells, osteoblast cells, cartilage cells, skeletal muscle cells, immortalized cells, cancer cells, keratinized cells, embryonic stem cells (ES cells), EBV transformed B cells, and induced pluripotent stem cells (iPS cells).

The cells may be primary cells in culture or a subcultured cell line, but are preferably primary cells in culture. Frozen or re-frozen cells may be used.

The cells may be cells that are two-dimensionally cultured or cells that are three-dimensionally cultured. The cells include spheroid obtained by culturing cells. Preferably, the cells are cells that are three-dimensionally cultured.

The cells are preferably cells having a high oxygen demanding property, such as cells forming skins, kidneys, livers, brains, neural tissues, cardiac muscle tissue or skeletal muscle tissues, cancer stem cells, and cancer cells. The cells are preferably, among the cells mentioned above, cells forming skins, kidneys, livers, brains, neural tissues, cardiac muscle tissue or skeletal muscle tissues, and cancer cells because they are cells having a high oxygen demanding property.

In an aspect of the present invention, the object to be cultured is more preferably a neural cell or a cardiac muscle cell, and still more preferably a neural cell. The object to be cultured is preferably a neural cell because oxygen can be sufficiently supplied in culture, which is suitable for three-dimensionally controlling the growth of an axon bundle of the neural cell.

In a culture vessel according to an aspect of the present invention, it is preferable that the first through-hole be a first chamber portion that receives the object to be cultured, the first chamber portion receive a cell body of a neural cell, and the space α receive an axon bundle extending from the cell body.

A tissue in an aspect of the present invention means similar cells aggregated to play the same role. The tissue is not limited, and examples thereof include epithelial tissues, connective tissues, muscle tissues, and neural tissues.

An organ in an aspect of the present invention means the tissues aggregated to perform cooperative work with a purpose. The organ is not limited, and is, for example, a lung, a heart, a liver, a kidney, a spleen, a pancreas, a gallbladder, an esophagus, a stomach, a skin, or a brain.

One of the objects to be cultured may be cultured singly, or two or more thereof may be cultured in combination.

In an aspect of the present invention, the term "culture" is used in a broad sense including not only proliferation and maintenance of cells, for example, but also processes such as seeding, subculture, induction of differentiation and induction of self-organization of cells, for example. A culture medium, for example, for use in culture is not limited, and a culture medium corresponding to the characteristics of cells, for example, may be selected.

### <Culture method>

A culture method according to an aspect of the present invention comprises a step (I) of incubating a cell, tissue or organ in a culture vessel which includes a vessel member (B) having an inverted recess portion on a lower surface of a bottom portion, and a first through-hole at one end of the inverted recess portion, and a film member (A) having oxygen permeability, wherein the film member (A) and the vessel member (B) are peelably bonded, the inverted recess portion and the film member (A) form a space α in a state where the film member (A) and the vessel member (B) are bonded, the first through-hole located at one end of the space α receives the object to be cultured, and the space α is a part of the culture space.

In a culture method according to an aspect of the present invention, the cell, tissue or organ is more preferably a neural cell or a cardiac muscle cell, and still more preferably a neural cell.

In a culture method according to an aspect of the present invention, the cell, tissue or organ is preferably a neural cell because the culture method is suitable for three-dimensionally controlling the growth of an axon bundle of a neural cell, and enables the axon bundle to be taken out without damage.

A culture method according to an aspect of the present invention preferably comprises a peeling step (II) of peeling a film member (A) from a vessel member (B) after the step (I), and a taking-out step (III) of taking out the incubated cell, tissue or organ after the peeling step (II).

It is preferable to carry out the peeling step (II) with the film member (A) kept horizontal for taking out the incubated cells, for example, without damage.

### <Method for producing culture vessel>

A method for producing a culture vessel according to an aspect of the present invention comprises a bonding step of peelably bonding a vessel member (B) having an inverted recess portion on a back surface of a bottom portion and a first through-hole at one end part of the inverted recess portion, and a film member (A) having oxygen permeability.

A production method according to an aspect of the present invention enables production of a culture vessel in which in a state where a film member (A) and a vessel member (B) are bonded, the inverted recess portion and the film member (A) form a space α, the first through-hole located at one end of the space α receives the object to be cultured, and the space α is a part of the culture space.

### (Bonding step)

In a method for producing a culture vessel according to an aspect of the present invention, the bonding step is not particularly limited, and can be carried out by, for example, hot-pressing and laser irradiation.

In a production method according to an aspect of the present invention, the bonding step is preferably carried out by hot-pressing because the members are peelably bonded easily.

In the case where the bonding step is carried out by hot-pressing, the bonding step is carried out using a hot-press molding machine. There is no limitation on hot-pressing conditions. It is preferable to perform heating at 20 to 200°C and pressing for 1 second to 10 minutes, and it is more preferable to perform heating at 50 to 150°C and pressing for 5 seconds to 1 minute.

In a method for producing a culture vessel according to an aspect of the present invention, the bonding step preferably comprises a step of performing hydrophilization treatment on a part or a whole of the bonded surface of a film member (A) and a vessel member (B), and then hot-pressing the hydrophilization-treated bonded surface, or a laser irradiation step of performing laser irradiation in a state where the bonded surface of the film member (A) and the bonded surface of the vessel member (B) are in contact with each other.

In an aspect of the present invention, the bonded surface refers to a surface at which the film member (A) and the vessel member (B) are bonded.

More specifically, in the film member (A), a part of the bonded surface refers to a surface forming the space α that is the upper surface of the film member (A). In addition, the whole of the bonded surface refers to the upper surface of the film member (A).

In the vessel member (B), a part of the bonded surface refers to a surface forming the space α that is an inverted recess portion of the lower surface of the bottom portion of the vessel member (B). In addition, the whole of the bonded surface refers to the lower surface of the bottom portion of the vessel member (B).

As a bonding step for a culture vessel according to an aspect of the present invention, a step of performing hydrophilization treatment on the whole of the bonded surface of the film member (A) by vacuum plasma treatment, performing hydrophilization treatment on the whole of the bonded surface of the vessel member (B) by normal-pressure plasma treatment, and then hot-pressing the hydrophilization-treated bonded surfaces is more preferably carried out.

### Examples

Hereinafter, an aspect of the present invention will be described in further detail with reference to Examples, but the present invention is not limited to these Examples.

### (Measurement)

A method for measuring an oxygen permeation coefficient, a method for calculating an oxygen permeability, a method for measuring a peel strength, a method for measuring total light transmittance, measurement of a glass transition point, and a method for measuring a water contact angle in Examples are

### described below.

### [Method for measuring oxygen permeation coefficient and method for calculating oxygen permeability]

For the oxygen permeability, the oxygen permeation coefficient of a measurement sample was measured in an environment at a temperature of 23°C and a humidity of 0% in accordance with Japanese Industrial Standard JIS-K-7126-1 using a differential pressure type gas permeation rate measurement apparatus MT-C3 manufactured by Toyo Seiki Seisaku-sho, Ltd. The diameter of a measurement portion was 70 mm (the permeation area was 38.46 cm²). Since the oxygen permeation coefficient was thought to be high, the actual permeation area was set to 5.0 cm² by applying an aluminum mask to the sample in advance.

The value of the oxygen permeation coefficient [cm³·mm/(m²·24h·atm)] measured was divided by the thickness (µm) of a film member (A) or a cover slip to calculate the oxygen permeability [cm³/(m²·24h·atm)].

### [Measurement of peel strength]

For the peel strength, the 90° peel strength was measured in accordance with Japanese Industrial Standard JIS-K 6854-1: 1999 (adhesive-test method for bonding strength against peeling, 90 degrees peeling) using a tensile testing machine (Model SVZ-500 NB manufactured by IMADA-SS Corporation).

### [Measurement of total light transmittance]

For the measurement sample, the total light transmittance was measured in accordance with Japanese Industrial Standard JIS K 7361-1 using a hazemeter (NDH2000 manufactured by NIPPON DENSHOKU INDUSTRIES Co., Ltd. Co., Ltd.) .

### [Measurement of glass transition point]

The glass transition point was measured in accordance with Japanese Industrial Standard JIS-K7121: 1987 (Measurement method for transition temperature of plastics) using a differential scanning calorimeter.

### [Measurement of water contact angle]

The water contact angle after hydrophilization of the culture surface was measured in accordance with Japanese Industrial Standard JIS-R3257 (Testing method of wettability of glass substrate). The measurement can be performed by a method in which water droplet with a volume of 4 µL or less which can be considered to have a spherical shape under constant temperature and humidity conditions of 25 ± 5°C and 50 ± 10% is dropped to a surface of a measurement sample, and by a static drop method, the angle of a contact interface between the substrate and the water droplet within 1 minute immediately after the water droplet comes into contact with the surface of the measurement sample is measured.

In the present embodiment, a numerical value within 1 minute immediately after the contact of the water droplet in the above-described method was taken as a property value.

### [Production Example 1] Production of films (A-1) and (A-2)

A 4-methyl-1-pentene polymer (trade name: TPX, manufactured by Mitsui Chemicals, Inc., total light transmittance 92%, glass transition point: 40°C, weight-average molecular weight (Mw): 42.8 × 10⁴) was provided, and put in an extrusion machine with a T-die which is equipped with a full flight-type screw that extrudes a substrate layer, the extrusion temperature and the roll temperature were set to 270°C and 60°C, respectively, and extrusion molding was performed while the roll rotation speed condition was changed, thereby obtaining two films that differ in thickness. A film having a thickness of 50 µm was a film (A-1), and a film having a thickness of 100 µm was a film (A-2).

In Examples, the film (A-1) and the film (A-2) were used as the film member (A). In addition, in Comparative Example, a cover slip was used instead of the film member (A).

Film (A-1): A 4-methyl-1-pentene polymer film having a thickness of 50 µm was used. The content of the 4-methyl-1-pentene polymer (X) was 99.5% by mass in 100% by mass of the film (A-1). The content of constitutional units derived from 4-methyl-1-pentene was 98.4 mol% based on the amount of all repeating constitutional units of the 4-methyl-1-pentene polymer (X), which is defined as 100 mol%.

Film (A-2): A 4-methyl-1-pentene polymer film having a thickness of 100 µm was used. The content of the 4-methyl-1-pentene polymer (X) was 99.5% by mass in 100% by mass of the film (A-1). The content of constitutional units derived from 4-methyl-1-pentene was 98.4 mol% based on the amount of all repeating constitutional units of the 4-methyl-1-pentene polymer (X), which is defined as 100 mol%.

Cover slip: Borosilicate glass having a thickness of 1,000 µm (manufactured by Matsunami Glass Ind. Ltd., trade name: FROST SLIDE GLASS S2112) was used.

Table 1 shows the materials, thickness (um), oxygen permeation coefficients (cm³·mm/m²·24 h·atm) and oxygen permeability (cm³/m²·24 h·atm) of the film (A-1), the film (A-2) and the cover slip.

**[Table 1]**

| | Material | Thickness (µm) | Oxygen permeation coefficient (cm³·mm/m²·24h·atm) | Oxygen permeability (cm³/m²·24h·atm) |
|---|---|---|---|---|
| Film (A-1) | 4-Methyl-1-pentene polymer | 50 | 1912 | 38240 |
| Film (A-2) | 4-Methyl-1-pentene polymer | 100 | 1900 | 19000 |

| | | | | |
|---|---|---|---|---|
| Cover slip | Borosilicate glass | 1000 | 0.001 | 0.001 |

### [Production Example 2] Production of vessel members (B-1) and (B-2)

A Vessel member (B) was produced by an injection molding method using a 4-methyl-1-pentene polymer (trade name: TPX, manufactured by Mitsui Chemicals, Inc., total light transmittance 92%, glass transition point: 30°C) or polystyrene (trade name: TOYO STYROL GP MW1D, manufactured by TOYO STYRENE Co., Ltd, total light transmittance 90%, glass transition point: 100°C) as a raw material.

A vessel member produced using a 4-methyl-1-pentene polymer as a raw material was a vessel member (B-1), and a vessel member produced using polystyrene as a raw material was a vessel member (B-2).

The shapes of the vessel members (B-1) and (B-2) are as in Figure 1. In the vessel member (B), the upper surface was open, and the bottom surface had two cylindrical first through-holes having a diameter of 1 mm, and two cylindrical second through-holes having a diameter of 0.5 mm. In a culture test described later, the cylindrical first through-hole having a diameter of 1 mm was used as a first chamber, and cylindrical second through-hole having a diameter of 0.5 mm was used as a second chamber.

The vessel members (B-1) and (B-2) had a size of 2.4 cm in length, 1.2 cm in width and 1.1 cm in height.

The lower surface of the bottom portion had two grooves having an inverted recess shape in cross-sectional view and communicating between one end of the first through-hole and one end of the second through-hole. The groove having an inverted recess shape was a groove having a semicircular shape in cross-sectional view, and had a diameter of 80 µm and a length of 15 mm. The groove is a portion which, when the film (A-1) or (A-2) is bonded in a step described later, forms a space α, which forms a channel.

There were two spaces α on the lower surface of the bottom portion of each of the vessel members (B-1) and (B-2).

The volume of the liquid storage portion of each of the vessel members (B-1) and (B-2) was 2.3 mL.

As Comparative Example, a vessel member made of polydimethylsiloxane (P-Chip manufactured by Jiksak Bioengineering, Inc.) was provided, and used as a vessel member (Comparative Example). The vessel member (Comparative Example) was identical in shape to the vessel members (B-1) and (B-2).

### [Production Example 3] Production of culture vessel (vacuum plasma treatment)

Hydrophilization treatment was performed on the whole of each of the film (A-1), the vessel member (B-1) and the vessel member (B-2). As the hydrophilization treatment, vacuum plasma treatment was performed.

Table 2 shows water contact angles (°) before and after the vacuum plasma treatment.

**[Table 2]**

| Plasma-treated member | Water contact angle (before vacuum plasma treatment) | Water contact angle (after vacuum plasma treatment) |
|---|---|---|
| Film (A-1) | 106° | 47° |
| Vessel member (B-1) | 103° | 39° |
| Vessel member (B-2) | 89° | 3° |

For the film (A-1), the vessel member (B-1) and the vessel member (B-2) to which vacuum plasma treatment had been applied, the film (A-1) was bonded to the lower surface of the bottom portion of each of the vessel member (B-1) and the vessel member (B-2) by hot-pressing to produce culture vessels 1 to 4. Table 3 show hot-pressing conditions for the culture vessels 1 to 4.

**[Table 3]**

| Culture vessel | Film member (A) | Vessel member(B) | Hot-pressing condition |
|---|---|---|---|
| Culture vessel 1 | Film (A-1) | Vessel member (B-1) | 100°C, 0.50 kN, 20 seconds |
| Culture vessel 2 | Film (A-1) | Vessel member (B-1) | 60°C, 0.75 kN, 60 seconds |
| Culture vessel 3 | Film (A-1) | Vessel member (B-1) | 70°C, 0.25 kN, 60 seconds |
| Culture vessel 4 | Film (A-1) | Vessel member (B-2) | 90°C, 2.0 kN, 60 seconds |

### [Production Example 4] Production of culture vessel (normal-pressure plasma treatment)

Hydrophilization treatment was performed on the whole of each of the film (A-1) and the film (A-2) using a normal-pressure plasma surface treatment apparatus (manufactured by Sekisui Chemical Company, Limited).

For the normal-pressure plasma treatment, corona treatment was performed using a table-type corona treatment apparatus (manufactured by Kasuga electric works Ltd.) (treatment rate: 3 m/min, power: 0.5 kW, two reciprocations). Table 4 shows water contact angles (°) of the film (A-1) and the film (A-2) to which normal-pressure plasma treatment was applied.

**[Table 4]**

| Culture vessel | Film member (A) | Film thickness | Water contact angle (after normal-pressure plasma treatment) | Vessel member (B) |
|---|---|---|---|---|
| Culture vessel 5 | Film (A-1) | 50 µm | 70° | Vessel member (B-1) |
| Culture vessel 6 | Film (A-2) | 100 µm | 70° | Vessel member (B-1) |

The film (A-1) or the film (A-2) to which normal-pressure plasma treatment had been applied was bonded to the lower surface of the bottom portion of the vessel member (B-1) by carbon dioxide laser beam irradiation to produce culture vessels 5 and 6. Normal-pressure plasma treatment was not applied to the vessel member (B-1).

The carbon dioxide laser beam irradiation for the culture vessels 5 and 6 was such that the film member (A) and the vessel member (B) were bonded by carbon dioxide laser under conditions of a wavelength of 10.6 µm, continuous-wave oscillation, and a beam diameter of about 2 mm, and carbon dioxide laser was applied from the film side with a power of 0.03 J/mm at a feed rate of 200 mm/sec to prepare the culture vessels 5 and 6.

As Comparative Example, a cover slip to which hydrophilization treatment had not been applied was bonded to the lower surface of the bottom portion of the vessel member (Comparative Example) with a commercially available sealing material to produce a culture vessel 7.

### [Example 1] Measurement of peel strength and occurrence or non-occurrence of damage

The peel strength of the culture vessels 1 to 7 was measured by the above-described measurement method.

Occurrence or non-occurrence of damage was observed for the film member and the vessel member after measurement of the peel strength. Table 5 shows results for the culture vessels 1 to 6 as Examples, and the culture vessel 7 as Comparative Example.

### [Example 2] Liquid leakage test

The liquid storage portion of each of the culture vessels 1 to 7 was filled with ethanol, followed by standing for 12 hours. Thereafter, occurrence or non-occurrence of liquid leakage on the bonded surface of the film member and the vessel member was visually observed. Table 5 shows results for the culture vessels 1 to 6 as Examples, and the culture vessel 7 as Comparative Example.

**[Table 5]**

| Culture vessel | | Peel strength | Damage of film member and vessel member after peel strength measurement | Liquid leakage test |
|---|---|---|---|---|
| Example | Culture vessel 1 | 0.7 N/cm | Both peeled without damage | No liquid leakage |
| | Culture vessel 2 | 0.6 N/cm | Both peeled without damage | No liquid leakage |
| | Culture vessel 3 | 0.4 N/cm | Both peeled without damage | No liquid leakage |
| | Culture vessel 4 | 1.0 N/cm | Both peeled without damage | No liquid leakage |
| | Culture vessel 5 | 0.8 N/cm | Both peeled without damage | No liquid leakage |
| | Culture vessel 6 | 0.8 N/cm | Both peeled without damage | No liquid leakage |
| Comparative Example | Culture vessel 7 | (measurement impossible) | Cover slip broken during peeling | No liquid leakage |

### [Example 3] Culture test

### (Preparation of motor nerve cell spheroid)

Motor nerve cells derived from human iPS cells (Elixirgen Scientific, Inc.) were seeded on a commercially available culture plate, and cultured at 37°C for 7 days in an atmosphere of 20% oxygen/5% carbon dioxide/75% nitrogen to prepare a spheroid 1.

### (Sterilization of vessel)

The liquid storage portion of each of the culture vessels 1 to 7 was filled with ethanol. The space α was confirmed to be filled with ethanol by visual observation through a film or a cover slip bonded to the lower surface of the bottom portion of the culture vessel.

The culture vessels 1 to 7 filled with ethanol were placed still at room temperature for 10 minutes. Thereafter, all the ethanol inside the culture vessel was discharged from the opening of the upper surface of the liquid storage portion, and air drying was performed at room temperature, followed by application of ultraviolet sterilization.

### (Coating treatment of culture surface)

Geltrex (manufactured by Thermo Fisher Scientific, Inc.) was dissolved in sterile water to prepare an aqueous solution for coating treatment. The liquid storage portion of each of the sterilized culture vessels 1 to 7 was filled with the aqueous solution for coating treatment. The space α was confirmed to be filled with the aqueous solution for coating treatment by visual observation through a film or a cover slip bonded to the lower surface of the bottom portion of the culture vessel.

The culture vessel was placed still at 37°C for 1 hour, and all the aqueous solution for coating treatment inside the culture vessel was then discharged from the opening of the upper surface of the liquid storage portion to apply coating treatment to the culture surface.

### (Preparation of axon bundle)

Neural cells (spheroid 1) were seeded in the first chamber portion of each of the culture vessels 1 to 7 to which surface treatment had been applied, and the liquid storage portion of each vessel was filled with a culture solution (manufactured by Thermo Fisher, Inc., trade name: Neurobasal Plus Medium). The space α being filled with the culture solution was confirmed by visual observation through a film or a cover slip bonded to the lower surface of the bottom portion of the culture vessel.

The cells were cultured at 37°C for 17 days in an atmosphere of 20% oxygen/5% carbon dioxide/75% nitrogen. For all of the culture vessels 1 to 7, axons being grown in a channel which is the space α were visually confirmed through the film or the cover slip from the lower surface of the bottom portion of the culture vessel.

An axon bundle was cultured in the channel of each of the culture vessels 1 to 7, and all the culture solution inside the culture vessel was then discharged from the opening of the upper surface of the liquid storage portion to obtain a culture vessel with neural cells including an axon bundle.

### (Taking-out of axon bundle)

For the culture vessels 1 to 6 of Examples, a film at the lower portion of the bottom surface of the vessel was peeled by the hand from the culture vessel with neural cells including an axon bundle, and neural cells including an axon bundle grown in the channel were collected with tweezers.

For the culture vessel 7 of Comparative Example, the axon bundle was taken out by injecting sterile water under pressure from the second chamber portion to discharge the axon bundle grown in the channel together with the sterile water from the first chamber side because it was not possible to peel the cover slit at the lower portion of the bottom surface of the vessel by the hand, and neural cells including an axon bundle were collected with tweezers.

### (Evaluation of neural cells after culture)

The neural cells (spheroid 1) including an axon bundle and collected from the culture vessels 1 to 7 were observed with an optical microscope. Specifically, the neural cells were observed with an optical microscope through the film bonded to the lower portion of the bottom surface of the culture vessel. The results of observation with the culture vessels 1, 3 and 4 are shown in Figure 3.

Culture using the culture vessels 1, 3 and 4 resulted in growth of the axon bundle to a length of 15 mm, 15 mm and 10 mm, respectively.

The neural cells cultured with the culture vessels 1 to 6 were cultured in a form similar to that of tissues in the living body by three-dimensionally controlling the growth.

The neural cells cultured with the culture vessel 7, whose growth was three-dimensionally controlled, nevertheless underwent distortion of the axon and formation of bumps, and were not cultured in a form similar to that of tissues in the living body.

### (Evaluation of occurrence or non-occurrence of damage of axon bundle)

The neural cells (spheroid 1) including an axon bundle and collected from the culture vessels 1, 3, 5 and 7 were observed with an optical microscope to confirm occurrence or non-occurrence of damage of the axon bundle.

When damages such as cuts and breaks were unlikely to be observed in the axon bundle, it was evaluated that "damage did not occur". When damages such as cuts and breaks were clearly confirmed in the axon bundle, it was evaluated that "damage occurred".

For each of the culture vessels 1, 3, 5 and 7, culture was performed 10 times under the same condition, and neural cells (spheroid 1) including collected axon bundles were observed. Table 6 shows the results.

**[Table 6]**

| | | Number of cultures without damage in collection | Number of cultures with damage |
|---|---|---|---|
| Example | Culture vessel 1 | 10 | 0 |
| | Culture vessel 3 | 10 | 0 |
| | Culture vessel 5 | 10 | 0 |
| Comparative Example | Culture vessel 7 | 7 | 3 |

These results revealed that for culture vessels 1, 3 and 5, the film was peeled, and axon bundles were taken out without damage, but for the culture vessel 7, it was not possible to peel the cover slip, and thus a certain proportion of axon bundles were damaged.

In the culture vessels 1 to 6, sufficient oxygen for growth is supplied from the bottom portion of the culture vessel, to which neural cells attached, and there was a culture space suitable for three-dimensionally controlling the growth of neural cells, so that it was possible to culture the neural cells in a form similar to that of tissues in the living body.

Further, the axon bundles of the neural cells cultured in the culture vessels 1, 3 and 5 were taken out without damage by peeling the film member (A) from the vessel member (B).

On the other hand, in the culture vessel 7, sufficient oxygen for growth was not supplied from the bottom portion of the culture vessel, to which neural cells attached. Therefore, the axon distorted, and bumps were formed. Thus, it was not possible to culture neural cells in a form similar to that of tissues in the living body.

In addition, for the axon bundles of the neural cells cultured with the culture vessel 7, the axon bundle had damages such as cuts or breaks because it was not possible to peel the film member (A) from the vessel member (B), and sterile water was injected under pressure from the first chamber portion many times to take out the axon bundle.

### Reference Signs List

1: Film member (A)
2: Vessel member (B)
10: First through-hole
11: First chamber portion
20: Second through-hole
21: Second chamber portion
30: Liquid storage portion
40: Inverted recess portion
50: Space α
60: Cell body of neural cell (spheroid 1)
70: Axon bundle extending from cell body of neural cell (spheroid 1)

## Claims

1. A culture vessel having a culture space for culturing an object to be cultured,
the culture vessel comprising:
a vessel member (B) having an inverted recess portion on a lower surface of a bottom portion, and a first through-hole at one end of the inverted recess portion; and
a film member (A) having oxygen permeability,
wherein the film member (A) and the vessel member (B) are peelably bonded,
the inverted recess portion and the film member (A) form a space α in a state where the film member (A) and the vessel member (B) are bonded,
the first through-hole located at one end of the space α receives the object to be cultured, and
the space α is a part of the culture space.

2. The culture vessel according to claim 1, wherein the space α is a channel.

3. The culture vessel according to claim 2, wherein a width of the channel is within a range of 1 µm to 1,000 µm, and a depth of the channel is within a range of 1 µm to 1,000 µm.

4. The culture vessel according to claim 2 or 3, wherein a length of the channel is within a range of 1 µm to 100 µm.

5. The culture vessel according to claim 1 or 2, wherein a thickness of the film member (A) is within a range of 1 µm to 1,000 µm.

6. The culture vessel according to claim 1 or 2, wherein the inverted recess portion is an inverted recess portion for a channel.

7. The culture vessel according to claim 1 or 2, wherein a peel strength between the film member (A) and the vessel member (B) in a 90° peel test is within a range of 0.01 to 3.0 N/cm as measured in accordance with-K 6854-1: 1999.

8. The culture vessel according to claim 1 or 2, wherein a total light transmittance of the film member (A) is 80% or more as measured in accordance with-K-7361-1.

9. The culture vessel according to claim 1 or 2, wherein an oxygen permeability of the film member (A) at a temperature of 23°C and a humidity of 0% is within a range of 4,500 to 90,000 cm³/(m²·24h·atm).

10. The culture vessel according to claim 1 or 2, wherein the vessel member (B) contains a resin.

11. The culture vessel according to claim 10, wherein the resin is a thermoplastic resin.

12. The culture vessel according to claim 11, wherein a glass transition point of the thermoplastic resin is 120°C or lower as measured in accordance with-K 7121: 1987.

13. The culture vessel according to any one of claim 1 or 2, wherein the film member (A) contains a 4-methyl-1-pentene polymer (X).

14. The culture vessel according to any one of claim 1 or 2, wherein the vessel member (B) contains a 4-methyl-1-pentene polymer (X).

15. The culture vessel according to claim 13 or 14, wherein the 4-methyl-1-pentene polymer (X) is at least one polymer selected from a 4-methyl-1-pentene homopolymer (X1) and a copolymer (X2) of 4-methyl-1-pentene and at least one olefin selected from ethylene, propylene, and an α-olefin having 4 to 20 carbon atoms.

16. The culture vessel according to claim 1 or 2, wherein a water contact angle of at least a culture surface of any of the film member (A) and the vessel member (B) is 50° to 100°.

17. The culture vessel according to claim 1 or .2, wherein at least a culture surface of any of the film member (A) and the vessel member (B) is a culture surface coated with at least one material selected from the group consisting of a natural polymer material, a synthetic polymer material and an inorganic material.

18. The culture vessel according to claim 1 or 2, wherein at least a culture surface of any of the film member (A) and the vessel member (B) is a hydrophilization-treated culture surface.

19. The culture vessel according to claim 1 or 2, wherein a second through-hole is provided at the other end of the inverted recess portion, and the other end of the space α communicates with the second through-hole.

20. The culture vessel according to claim 19, wherein the vessel member (B) includes a liquid storage portion that stores a culture medium, and the liquid storage portion, the first through-hole, the second through-hole and the space α communicate.

21. The culture vessel according to claim 1 or 2, wherein the object to be cultured is a neural cell or a cardiac muscle cell.

22. The culture vessel according to claim 21,
wherein the first through-hole is a first chamber portion that receives the object to be cultured, and
the first chamber portion receives a cell body of the neural cell, and the space α receives an axon bundle extending from the cell body.

23. A method for culturing a cell, tissue or organ, comprising a step (I) of incubating a cell, tissue or organ in the culture vessel according to claim 1 or 2.

24. The culture method according to claim 23, comprising:
a peeling step (II) of peeling the film member (A) from the vessel member (B) after the step (I); and
a taking-out step (III) of taking out the incubated cell, tissue or organ after the peeling step (II).

25. A method for producing the culture vessel according to claim 1 or 2, comprising a bonding step of peelably bonding
a vessel member (B) having an inverted recess portion on a lower surface of a bottom portion, and a first through-hole at one end of the inverted recess portion, and
a film member (A) having oxygen permeability.

26. The method for producing a culture vessel according to claim 25, wherein the bonding step comprises
a step of performing hydrophilization treatment on a part or a whole of a bonded surface of the film member (A) and the vessel member (B), and then hot-pressing the hydrophilization-treated bonded surface, or
a laser irradiation step of performing laser irradiation in a state where the bonded surface of the film member (A) and the bonded surface of the vessel member (B) are in contact with each other.
